Europäisches Patentamt

European Patent Office

Office européen des brevets

(19)

(11) Veröffentlichungsnummer: **0 288 747 B1**

(12) # EUROPÄISCHE PATENTSCHRIFT

(45) Veröffentlichungstag der Patentschrift: **29.05.91**

(51) Int. Cl.⁵: **C07C 59/08**

(21) Anmeldenummer: **88104745.0**

(22) Anmeldetag: **24.03.88**

(54) **Calciumlactat-Glycerin-Addukt, Verfahren zu seiner Herstellung sowie seine Verwendung.**

(30) Priorität: **27.03.87 DE 3710177**

(43) Veröffentlichungstag der Anmeldung:
**02.11.88 Patentblatt 88/44**

(45) Bekanntmachung des Hinweises auf die
Patenterteilung:
**29.05.91 Patentblatt 91/22**

(84) Benannte Vertragsstaaten:
**AT BE CH DE ES FR GB GR IT LI LU NL SE**

(56) Entgegenhaltungen:
**DE-B- 331 695**

(73) Patentinhaber: **HOECHST AKTIENGESELL-
SCHAFT
Postfach 80 03 20
W-6230 Frankfurt am Main 80(DE)**

(72) Erfinder: **Reul, Bernhard
An den Geierwiesen 20
W-6240 Königstein/Taunus(DE)**
Erfinder: **Petri, Walter, Dr.
Panoramastrasse 19
W-6272 Niedernhausen/Taunus(DE)**

**Beschreibung**

Die Erfindung betrifft Calciumlactat-Glycerin-Addukt, ein festes, pulverförmiges, kristallines Reaktionsprodukt zwischen Calciumlactat und Glycerin und ein Verfahren zur Herstellung dieses neuen Stoffes. Die Erfindung betrifft ferner die Verwendung des Adduktes in der Pharmazie, Kosmetik und in Lebensmitteln.

Calciumlactat-Glycerin-Addukt ist eine Molekülverbindung zwischen 1 Mol Calciumlactat und 2 Mol Glycerin der Formel I

$$(CH_3\text{-}CHOH\text{-}COO)_2\ Ca \times 2\ H_2COH\text{-}HCOH\text{-}H_2COH \quad I$$

Das Verfahren zur Herstellung ist dadurch gekennzeichnet, daß man Calciumlactat mit wasserfreiem Glycerin im Molverhältnis von 1 zu mindestens 2 unter Erwärmung in einem wasserfreien $C_1$- bis $C_3$-Alkohol umsetzt, die erhaltene klare Lösung abkühlt und das Calciumlactat-Glycerin-Addukt abtrennt.

Das Calciumlactat wird zweckmäßigerweise entweder in Form der Hydrate, z.B. des 5-Hydrat, oder in wasserfreier Form eingesetzt. Bei der Umsetzung versetzt man 1 Mol Calciumlactat mit mindestens 2 Mol, vorzugsweise 4 Mol, Glycerin. Das wasserfreie Glycerin wird vorzugsweise mit wasserfreiem Methanol gemischt, wobei vorzugsweise Mischungen von 1 - 5 Gew.- Teilen, insbesondere 2 Teilen Methanol, mit 1 - 7, insbesondere 3 Gew.- Teilen, Glycerin eingesetzt werden. Das Reaktionsgemisch wird bis zum Kochen am Rückfluß erhitzt;

es entsteht eine klare Lösung, aus der beim Abkühlen das Addukt in Form von körnigen Kristallen ausfällt. Zur vollständigen Kristallisation kann die Löslichkeit des Adduktes im Reatkionsgemisch Glycerin/Methanol durch Zugabe von Aceton erniedrigt werden (gleiche Gew.- Teile Aceton bezogen auf das eingesetzte Glycerin). Die Substanz wird mit einem Gemisch Methanol/Aceton zur vollständigen Glycerinentfernung gewaschen. Die Abtrennung des Adduktes erfolgt z.B. durch Filtration.

Das Produkt ist weiß, fest, kristallin, feinpulvrig, freifließend, rieselfähig und physiologisch verträglich.

Es war überraschend, daß die neue Substanz trotz des hohen Glyceringehaltes (45,7 % G/G) wesentlich weniger hygroskopisch ist als wasserfreies Glycerin.

Ein weiterer Vorteil ist, daß die neue Substanz eine wesentlich höhere Kurzzeitlöslichkeit als Calciumlactat besitzt, wie die folgende Gegenüberstellung zeigt:

Löslichkeit in Wasser bei Raumtemperatur unter konstanten Bedingungen

| Löslichkeit nach (Min.) | Calciumlactat x 5 $H_2O$, M=308 | Calciumlactat-Glycerin-Addukt M=402 |
|---|---|---|
| 5 Min. | 7,7%(G/G)=1% Ca | 17,0%(G/G)=1,70% Ca |
| 15 Min. | -- | 17,0%(G/G)=1,70% Ca |
| 1440 Min. | 7,7%(G/G)=1% Ca | 9,8%(G/G)=0,98% Ca |

**(G/G= Gewichts-%)**

Die geringe Wasserdampfsorption und die hohe Löslichkeit von Calciumlactat-Glycerin-Addukt verbunden mit den anderen angegebenen Eigenschaften machen die neue Substanz als Hilfsstoff in allen den Fällen geeignet, in denen Calcium bei guter Löslichkeit und guter Verarbeitbarkeit benötigt wird, wie z.B. in der Pharmazie, Kosmetik oder auch in der Lebensmittelherstellung./

Die neue Substanz ist geschmacksneutral, so daß sie auch zur oralen Calciumtherapie eingesetzt werden kann. Hierzu eignen sich alle oralen Zubereitungsformen. Ihre Herstellung erfolgt nach bekannten Verfahren, d.h. das pulverförmige, kristalline Calciumlactat-Glycerin-Addukt wird mit den für die jeweilige Zubereitungsform üblichen Hilfsstoffen gemischt, ggf. granuliert und dann beispielsweise zu Tabletten, Trockensäften, wassersuspendierbaren Pulvern, Granulaten, Hartgelatinekapseln und Weichgelatinekapseln verarbeitet.

Das Addukt kann auch als Calciumspender in Nähr- und Stärkungsmittel eingearbeitet werden. Hierzu eignet sich das Addukt an sich oder das Addukt in Granulatform. Weiterhin besitzt die neue Substanz gute Hautverträglichkeit, so daß sie auch zur dermalen Calciumtherapie eingesetzt werden kann. Hierzu eignen sich vorzugsweise die wasserfreien dermalen Zubereitungsformen, wie beispielsweise Salben und Puder. Für diese Anwendungsform wird das pulverförmige, kristalline Calciumlactat-Glycerin-Addukt mikronisiert und dann nach bekannten Verfahren in Salben- und Pudergrundlagen eingearbeitet.

Neben diesen auf dem Gebiet der Nähr- und Stärkungsmittel und der Calciumtherapie liegenden Einsatzmöglichkeiten ist Calciumlactat-Glycerin-Adukt aufgrund seiner physikalischen Eigenschaften und technologischen Besonderheiten auch als Tablettierhilfsstoff einsetzbar, z.B. zur Förderung der Kompaktierung anstelle von Milchzucker oder auch als Füllstoff.

Eine besondere Verwendung findet die neue Substanz aufgrund ihrer guten physiologischen Verträglichkeit und infolge ihrer günstigen Lösungseigenschaft in festen Drug Delivery Systems (DDS), z.B. in Formlingen, Extrudaten, Pellets oder Mikrokapseln, indem Calciumlactat-Glycerin-Adukt die Freigabe der eingearbeiteten Wirkstoffe kontrolliert. Zur Herstellung der Drug Delivery Systems kommen die entsprechenden bekannten Verfahren zur Anwendung.

Aus pharmazeutisch-technologischer Sicht und aus Sicht der Calciumtherapie ist die neue Calciumverbindung, Calciumlactat-Glycerin-Adukt, als spezieller Hilfsstoff für Pharmazie und Kosmetik ein deutlicher Fortschritt gegenüber dem Stand der Technik aufgrund seiner besonderen physikalischen Eigenschaften bei gleichzeitig guter physiologischer Verträglichkeit.

## Herstellungsbeispiele

**Beispiel 1** 30,8 g Calciumlactat x 5 $H_2O$ werden in einer Lösung von 92,0 g Glycerin wasserfrei und 76,2 ml Methanol (wasserfrei) 10 Min. lang unter Rückfluß erhitzt. Die klare Lösung wird rasch (5 Min.) auf Raumtemperatur abgekühlt und mit 115 ml Aceton versetzt. Nach 24-stündigem Rühren werden die gebildeten Kristalle abgesaugt und bei Raumtemperatur im Vakuum 24 Stunden lang getrocknet.
Ausbeute: 37,6 g = 93 % der Theorie

**Beispiel 2** 30,8 g Calciumlactat x 5 $H_2O$ werden in einer Lösung von 92,0 g Glycerin wasserfrei und 76,2 ml Methanol (wasserfrei) unter Rühren 20 Min. lang auf 50°C erhitzt. Die klare Lösung wird langsam (2 Std.) unter Rühren auf Raumtemperatur abgekühlt, angeimpft und weitere 24 Std. bei Raumtemperatur gerührt. Der entstandene Kristallbrei wird abgedrückt (2,5 bar), der Filterkuchen mit 50 ml eines Aceton/Methanol-Gemisches (3 : 1 Gew.-Teile) gewaschen und anschließend 24 Std. bei Raumtemperatur im Vakuum getrocknet.
Ausbeute: 30,6 g = 76 % der Theorie * Gew.-Teile

## Analyse des Reaktionsproduktes:

|  | Analysenergebnis | theoretischer Wert |
|---|---|---|
| **Calcium** | 9,70 % | 9,95 % |
| **Lactat** | 43,70 % | 44,28 % |
| **Glycerin** | 43,70 % | 45,70 % |

## Ansprüche

1. Calciumlactat-Glycerin-Adukt der Formel I

$(CH_3\text{-}CHOH\text{-}COO)_2$ Ca x 2 $H_2COH\text{-}HCOH\text{-}H_2COH$   I

**2.** Verfahren zu Herstellung des Calciumlactat-Glycerin-Adduktes gemäß Anspruch 1, dadurch gekennzeichnet, daß man Calciumlactat mit wasserfreiem Glycerin im Molverhältnis von 1 zu mindestens 2 unter Erwärmen in einem wasserfreien $C_1$-bis $C_3$-Alkohol umsetzt, die erhaltene klare Lösung abkühlt und das Calciumlactat-Glycerin-Addukt abtrennt.

**3.** Verwendung des Calciumlactat-Glycerin-Adduktes gemäß Anspruch 1 als Calciumspender in der Pharmazie, Kosmetik und Lebensmitteln.

**4.** Verwendung des Calciumlactat-Glycerin-Adduktes gemäß Anspruch 1 als Hilfstoff in der Pharmazie und Kosmetik.

Patentansprüche für den folgenden Vertragsstaat: ES

**1.** Verfahren zur Herstellung von Calcium actat-Glycerin-Addukt der Formel I

$$(CH_3\text{-}CHOH\text{-}COO)_2 \; Ca \times 2 \; H_2COH\text{-}HCOH\text{-}H_2COH \quad I$$

dadurch gekennzeichnet, daß man Calciumlactat mit wasserfreiem Glycerin im Molverhältnis von 1 zu mindestens 2 unter Erwärmen in einem wasserfreien $C_1$ bis $C_3$-Alkohol umsetzt, die erhaltene klare Lösung abkühlt und das Calciumlactat-Glycerin-Addukt abtrennt.

**2.** Verwendung des Calciumlactat-Glycerin-Adduktes erhältlich nach Anspruch 1 als Calciumspender in der Pharmazie, Kosmetik und in Lebensmitteln.

**3.** Verwendung des Calciumlactat-Glycerin-Adduktes erhältlich nach Anspruch 1 als Hilfstoff in der Pharmazie und Kosmetik.

## Claims

**1.** Calcium lactate-glycerol adduct of the formula I

$$(CH_3\text{-}CHOH\text{-}COO)_2 \; Ca \times 2 \; H_2COH\text{-}HCOH\text{-}H_2COH \quad I$$

**2.** A process for the preparation of the calcium lactate-glycerol adduct as claimed in claim 1, which comprises reacting calcium lactate with anhydrous glycerol in a molar ratio of 1 to at least 2, while warming in an anhydrous $C_1$- to $C_3$-alcohol, cooling the resulting clear solution and separating off the calcium lactate-glycerol adduct.

**3.** The use of the calcium lactate-glycerol adduct as claimed in claim 1 as a calcium donor in pharmacy, cosmetics and foodstuffs.

**4.** The use of the calcium lactate-glycerol adduct as claimed in claim 1 as an auxiliary in pharmacy and cosmetics.

Claims for the following Contracting State: ES

**1.** A process for the preparation of a calcium lactate-glycerol adduct of the formula I

$$(CH_3) \text{-}CHOH\text{-}COO )2 \; Ca \times 2 \; H_2COH\text{-}HCOH\text{-}H_2C0H \quad I$$

which comprises reacting calcium lactate with anhydrous glycerol in a molar ratio of 1 to at least 2, while warming in an anhydrous $C_1$- to $C_3$-alcohol, cooling the resulting clear solution and separating off the calcium lactate-glycerol adduct.

**2.** The use of the calcium lactate-glycerol adduct obtainable as claimed in claim 1 as a calcium donor in pharmacy, cosmetics and foodstuffs.

3. The use of the calcium lactate-glycerol adduct obtainable as claimed in claim 1 as an auxiliary in pharmacy and cosmetics.

**Revendications**

1. Adduct de glycérol et de lactate de calcium de formule I

$$(CH_3\text{-}CHOH\text{-}COO)_2Ca \times 2H_2COH\text{-}HCOH\text{-}H_2COH \quad (I)$$

2. Procédé pour la préparation de l'adduct de glycérol et de lactate de calcium selon la revendication 1, caractérisé en ce que l'on fait réagir du lactate de calcium avec du glycérol anhydre, dans le rapport molaire de 1 à au moins 2, en chauffant dans un alcool en $C_1$-$C_3$ anhydre, on refroidit la solution limpide obtenue et on sépare l'adduct de glycérol et de lactate de calcium.

3. Utilisation de l'adduct de glycérol et de lactate de calcium selon la revendication 1, en tant que donneur de calcium dans l'industrie pharmaceutique, dans celle des cosmétiques et dans des produits alimentaires.

4. Utilisation de l'adduct de glycérol et de lactate de calcium selon la revendication 1, en tant qu'adjuvant dans l'industrie pharmaceutique et dans celle des cosmétiques.

Revendications pour l'Etat Contractant suivant: ES

1. Procédé pour la préparation de l'adduct de glycérol et de lactate de calcium, de formule I

$$(CH_3\text{-}CHOH\text{-}COO)_2Ca \times 2H_2COH\text{-}HCOH\text{-}H_2COH \quad (I) ,$$

caractérisé en ce que l'on fait réagir du lactate de calcium avec du glycérol anhydre dans le rapport molaire de 1 à au moins 2, en chauffant, dans un alcool en $C_1$-$C_3$ anhydre, on refroidit la solution limpide obtenue et on sépare l'adduct de glycérol et de lactate de calcium.

2. Utilisation de l'adduct de glycérol et de lactate de calcium préparable selon la revendication 1, en tant que donneur de calcium dans l'industrie pharmaceutique, dans celle des cosmétiques et dans des produits alimentaires.

3. Utilisation de l'adduct de glycérol et de lactate de calcium préparable selon la revendication 1, en tant qu'adjuvant dans l'industrie pharmaceutique et dans celle des cosmétiques.